# EUROPEAN PATENT APPLICATION

(11) **EP 4 763 192 A1**
(43) Date of publication of application: **24.06.2026**
(21) Application number: 25864512.6
(22) Date of filing: 04.07.2025
(51) Int. Cl.: A61K 9/06, A61K 47/36, A61K 31/353, A61K 31/355, A61K 47/22, A61P 3/04

(54) **HYDROGEL MICROSPHERES, PREPARATION METHOD THEREFOR, AND USE THEREOF**

(30) Priority: 26.09.2024 CN 202411351167
(71) Applicant: Novastra Therapeutics Inc., Dover, DE 19901 (US)
(72) Inventor: GUO, Junling, Chengdu, Sichuan 610041 (CN); WU, Yue, Chongqing 401434 (CN); HE, Yunxiang, Chengdu, Sichuan 610000 (CN); YANG, Lie, Chengdu, Sichuan 610041 (CN); MA, Qin, Chengdu, Sichuan 610041 (CN)
(74) Representative: Chung, Hoi Kan
(86) International application number: PCT/CN2025/107079
(87) International publication number: WO 2026/066491

(57) **Abstract**

The present disclosure relates to the technical field of hydrogels and discloses hydrogel microspheres, preparation methods, and applications thereof. Hydrophobic polyphenol-based micelles are formed through the self-assembly of epigallocatechin gallate (EGCG) and Vitamin E. These micelles, featuring EGCG at the core, form a stable structure via non-covalent interactions (e.g., hydrophobic interactions, hydrogen bonding, and π-π stacking), thereby enhancing the stability and functionality of the hydrophobic core. By incorporating this hydrophobic structure into a hydrophilic sodium alginate gel matrix, hydrogel microspheres with a unique amphiphilic heterogeneous structure are obtained. The hydrogel microspheres exhibit excellent stability in aqueous environments and within the gastrointestinal tract, enabling the active capture and sequestration of dietary fats, thereby significantly improving lipid-capturing efficiency.

## Description

### TECHNICAL FIELD

The present disclosure relates to the technical field of hydrogels, and more particularly to amphiphilic heterogeneous hydrogel microspheres, methods for their preparation, and their therapeutic applications in inhibiting intestinal absorption of dietary fats and treating obesity.

### BACKGROUND

Preventing intestinal absorption of dietary fat is a main determinant in treating obesity. In the small intestine, dietary fats are emulsified by bile acids and hydrolyzed by lipases to form micelles, which are transported across intestinal epithelial cells into the bloodstream for systemic utilization. Absorption occurring at the emulsion interface is critical. Various approaches have been proposed to block intestinal absorption of dietary fat and thereby restrain obesity, with lifestyle interventions and medical therapies (such as diet adjustment, anti-obesity drugs, and bariatric surgery) being widely adopted; however, these are limited by low patient compliance and multiple side effects. Thus, biomaterials, due to their efficiency, multifunctionality and biocompatibility, show promising prospects in addressing obesity.

Currently reported biomaterials targeting the intestinal absorption of dietary fat mainly include ionic liquids, sucrose esters, and dietary fibers. Ionic liquids and sucrose esters reduce fat absorption by forming a barrier between intestinal cells and dietary fat, but undigested fat remaining in the intestine may lead to steatorrhea. Dietary fibers (resistant starch and nanocellulose) can reduce such adverse intestinal effects and effectively reduce intestinal fat absorption due to their unique macromolecular complex structures; however, they lack dynamic, targeted fat-capturing ability because of their non-intelligent responsive structures.

### SUMMARY

To address the issues in the prior art, the present disclosure provides hydrogel microspheres, methods for their preparation, and therapeutic applications thereof. The hydrogel microspheres provided herein are heterogeneous hydrogels, which exhibit enhanced stability within the intestinal tract and are capable of dynamically capturing dietary fats.

In a first aspect, the present disclosure provides a method for preparing hydrogel microspheres, comprising the steps of:
self-assembling epigallocatechin gallate (EGCG) and Vitamin E in an ethanol solution to form hydrophobic polyphenol-based micelles;
uniformly mixing the hydrophobic polyphenol-based micelles with a sodium alginate solution to form a pre-gel; and
cross-linking the pre-gel with calcium ions, followed by washing and lyophilization.

In some embodiments, the mass ratio of the epigallocatechin gallate, Vitamin E, and sodium alginate is 1 : (0.7-0.9) : (0.6-1.2).

In some embodiments, the sodium alginate solution has a mass concentration of 1.5% to 3%.

In some embodiments, the lyophilization is performed at -80 °C for 36 to 48 hours.

In some embodiments, the concentration of the calcium ions is 1.5% to 2%.

In a second aspect, the present disclosure provides hydrogel microspheres prepared according to the method of the first aspect.

In a third aspect, the present disclosure provides a method for inhibiting intestinal absorption of dietary fats, comprising administering an effective amount of the hydrogel microspheres of the second aspect to a subject in need thereof.

In a fourth aspect, the present disclosure provides a method for treating obesity, comprising administering an effective amount of the hydrogel microspheres of the second aspect to a subject in need thereof.

Compared with the prior art, the present disclosure achieves the following technical effects: By self-assembling EGCG and Vitamin E into hydrophobic polyphenol-based micelles, a stable structure is formed via non-covalent interactions (such as hydrophobic interactions, hydrogen bonding, and π-π stacking) with EGCG at the core. This structure enhances the stability and functionality of the hydrophobic core. By subsequently incorporating this hydrophobic structure into a hydrophilic sodium alginate gel matrix, the resulting hydrogel microspheres possess a unique amphiphilic heterogeneous structure. These microspheres remain stable in aqueous environments and can actively and effectively capture dietary fats, thereby improving the lipid-capturing capacity and ensuring stability throughout the gastrointestinal tract.

### BRIEF DESCRIPTION OF THE DRAWINGS

FIG. 1 illustrates the demulsification effects of hydrogel microspheres with different ratios in Example 1, where (a) shows emulsion light transmittance and (b) shows the weight difference of the hydrogel microspheres before and after lipid absorption.
FIG. 2 illustrates characterization of the hydrogel microspheres in Example 1, where (a) shows UV spectra, (b) shows particle size distribution by dynamic light scattering (DLS), and (c) shows a transmission electron microscopy (TEM) image of the hydrophobic cavities.
FIG. 3 illustrates the amphiphilic properties of the hydrogel microspheres prepared in Example 2, where (a) shows the macroscopic morphology before and after lyophilization, (b) shows the amphiphilic interface captured via fluorescence microscopy, and (c) shows the amphiphilicity results via contact angle testing.
FIG. 4 illustrates structural characterization of the hydrogel microspheres prepared in Example 2, where (a) shows Fourier-transform infrared (FTIR) spectra, (b) shows elemental analysis, and (c) and (d) show thermogravimetric analysis (TGA) curves.
FIG. 5 illustrates the biocompatibility of the hydrogel microspheres prepared in Example 2, where (a) shows cytotoxicity data and (b) shows cell viability data.
FIG. 6 illustrates the gastrointestinal stability of the hydrogel microspheres prepared in Example 2, where (a) shows the EGCG/VE content in the supernatant after immersion in simulated gastrointestinal fluids, (b) shows the weight difference of the microspheres after immersion, and (c) shows the structural integrity of the microspheres after immersion.
FIG. 7 illustrates the dietary fat absorption capacity of the hydrogel microspheres prepared in Example 2, where (a) shows digital and fluorescence microscopy images of soybean oil, (b) shows the weight difference before and after lipid absorption, and (c) shows the absorption rates for various dietary lipids.
FIG. 8 illustrates (a) fluorescence imaging of the gastrointestinal tract of rats in Example 3, and (b) the levels of triglycerides and free fatty acids in the blood of the rats.
FIG. 9 is a statistical chart showing the food intake of various animal groups in Example 4.
FIG. 10 illustrates the efficacy of the hydrogel microspheres in obesity management in Example 4, where (a) shows body weight changes of rats in different treatment groups after 30 days of feeding, (b) shows the physical morphology of the rats, and (c) shows the weights of liver and adipose tissues.
FIG. 11 illustrates the in vitro fecal excretion of dietary fats facilitated by the hydrogel microspheres in Example 4, where (a) shows images of feces from each group, and (b) shows the fecal levels of triglycerides, cholesterol, free fatty acids, and bile acids (from left to right).
FIG. 12 illustrates the particle sizes of different micelles in the Comparative Example.

### DETAILED DESCRIPTION OF THE EMBODIMENTS

To clarify the objectives, technical solutions, and advantages of the present disclosure, the present disclosure is further described in detail below with reference to specific embodiments. It should be understood that these descriptions are merely exemplary and are not intended to limit the scope of the present disclosure. Furthermore, descriptions of well-known structures and techniques are omitted in the following description to avoid unnecessarily obscuring the concepts of the present disclosure. Where specific conditions are not specified in the examples, they are carried out according to conventional conditions or conditions recommended by the manufacturer. Reagents or instruments for which no manufacturer is specified are conventional products that can be obtained through commercial purchase.

A first embodiment of the present disclosure provides a method for preparing hydrogel microspheres, comprising:
self-assembling epigallocatechin gallate (EGCG) and Vitamin E in an ethanol solution to form hydrophobic polyphenol-based micelles;
uniformly mixing the hydrophobic polyphenol-based micelles with a sodium alginate solution to form a pre-gel; and
cross-linking the pre-gel with calcium ions, followed by washing and lyophilization.

In the embodiments of the present disclosure, by self-assembling epigallocatechin gallate (EGCG) and Vitamin E into hydrophobic polyphenol-based micelles, a stable structure is formed through non-covalent interactions (such as hydrophobic interactions, hydrogen bonding, and π-π stacking) with EGCG at the core, thereby enhancing the stability and functionality of the hydrophobic core. By subsequently mixing this hydrophobic structure with a hydrophilic sodium alginate gel, the resulting hydrogel microspheres possess a unique amphiphilic heterogeneous structure. These hydrogel microspheres skillfully combine hydrophobic and hydrophilic characteristics on the same plane, forming an integrated heterogeneous network. This allows the hydrogel microspheres to exist stably in an aqueous environment, actively capture dietary fats, improve the capacity for capturing dietary fats, and enhance the stability of the hydrogel microspheres in the gastrointestinal tract.

It should be noted that the EGCG component alone can only perform demulsification, and its binding force with dietary fats is insufficient, such that dietary fats are not easily retained within EGCG microspheres. During the implementation of the present disclosure, it was discovered that only the combination of EGCG with Vitamin E can undergo self-assembly to obtain hollow hydrophobic polyphenol-based micelles, which enhances the binding force with dietary fats and firmly locks the dietary fats inside the microspheres, thereby enhancing the functionality of the hydrophobic core. Subsequently, the hydrophobic polyphenol-based micelles are filled into a dietary matrix to construct the amphiphilic heterogeneous hydrogel microspheres.

It should further be noted that the heterogeneous structure hydrogel obtained by the present disclosure specifically refers to the formed amphiphilic interface structure, which is composed of different materials or different structures of the same material. These heterogeneous regions have different structures at the microscopic or macroscopic level, leading to different mechanical properties, water absorption performance, or biological activities. These heterogeneous hydrogels can simultaneously possess layered structures, encapsulated particles, or other types of composite structures. However, heterogeneous hydrogels in the prior art are mixtures containing different components, and the concept of "heterogeneity" is merely proposed in contrast to homogeneous hydrogels. Homogeneous hydrogels refer to hydrogels composed of a single type of polymer network whose chemical and physical properties are uniformly distributed throughout the system; in homogeneous hydrogels, polymer chains and cross-linking points are uniformly distributed on a macroscopic scale, meaning the same structure and composition will be found in any part of the hydrogel. Prior art heterogeneous hydrogels are usually constructed by adding different composite components for specific functions, mostly as heterogeneous multi-level structures.

The ethanol solution is used to dissolve Vitamin E and EGCG in an amount sufficient to fully dissolve them, and it is appropriate that the sodium alginate gel does not undergo agglomeration after adding sodium alginate. Those skilled in the art can select an appropriate amount of ethanol based on actual conditions.

In some preferred embodiments, the mass ratio of epigallocatechin gallate, Vitamin E, and sodium alginate is 1 : (0.7-0.9) : (0.6-1.2).

In some preferred embodiments, the mass concentration of the sodium alginate is 1.5-3%.

Regarding the conditions for lyophilization, no special limitation is required, and those skilled in the art can adaptively adjust them according to the required product performance. For example, in some preferred embodiments, the lyophilization is performed at -80 °C for 48 h.

In some preferred embodiments, to further increase the mechanical properties of the hydrogel, a calcium salt may also be added during the preparation of the pre-gel. After the subsequent cross-linking of the pre-gel with calcium ions, hydrochloric acid is added to release Ca²⁺ from the calcium salt, which cross-links with the hydrogel inside the microspheres, thereby improving the internal gelation of the microspheres. The calcium salt may be calcium sodium edetate (EDTA-CaNa₂).

A second embodiment of the present disclosure provides the hydrogel microspheres obtained according to the first embodiment.

The hydrogel microspheres of the embodiments of the present disclosure are hydrogels having an amphiphilic heterogeneous structure, which can enhance the adsorption capacity for dietary fats and achieve efficient capture of dietary fats.

A third embodiment of the present disclosure provides the use of the hydrogel microspheres of the second embodiment in the preparation of a medicament for preventing intestinal absorption of dietary fats.

The hydrogel microspheres of the embodiments of the present disclosure have a dual effect in preventing the intestinal absorption of dietary fats: first, the hydrophilic regions on the surface can contact emulsified lipid droplets to effectively capture them, laying the foundation for subsequent fat-locking behavior; second, the hydrophobic structure within the hydrogel microspheres can interact with the oil released by demulsification, firmly locking the oil within the spheres and excreting it from the body without causing adverse reactions such as steatorrhea. Driven by the combination of the interfacial activity of EGCG and the non-absorbability of the sodium alginate dietary matrix, the hydrogel microspheres can maintain their structural integrity in the gastrointestinal environment and safely encapsulate dietary fats inside the microspheres until natural excretion. Therefore, the hydrogel microspheres of the embodiments of the present disclosure can be applied to the preparation of medicaments for preventing intestinal absorption of dietary fats.

A fourth embodiment of the present disclosure provides the use of the hydrogel microspheres of the second embodiment in the preparation of a medicament for treating obesity.

The hydrogel microspheres of the embodiments of the present disclosure achieve the treatment of obesity by preventing the intestinal absorption of dietary fats, effectively avoiding common discomfort reactions in traditional anti-obesity biomaterial interventions, such as diarrhea, nausea, and bloating, and demonstrating great potential in obesity management. Therefore, the hydrogel microspheres of the embodiments of the present disclosure can be applied to the preparation of medicaments for treating obesity.

To make the technical solutions of the present disclosure clearer, the preparation and performance of the hydrogel microspheres are illustrated below through several specific examples.

### Example 1

### 1.1 Preparation of hydrogel microspheres

First, epigallocatechin gallate (EGCG) and Vitamin E (VE) were self-assembled in an ethanol solution to form hydrophobic polyphenol-based micelles (Fat lock). Then, the hydrophobic polyphenol-based micelles were added dropwise to a sodium alginate solution with a mass concentration of 2%, and stirred to form a uniform pre-gel. Hydrogel microspheres were formed via the Ca²⁺ ion cross-linking method. Finally, the microspheres were rinsed several times with distilled water to remove residual ions and lyophilized at -80 °C for 48 h to obtain amphiphilic hydrogel microspheres (Polyphenol-based fat trap, PFAT).

### 1.2 Effect of different raw material amounts on hydrogel microsphere performance

According to the method in 1.1, the mass ratios of sodium alginate:EGCG:VE were set to 0:0:0, 1:0:0, 1:1:0, 6:10:7, 6:10:9, 4:5:4, 12:10:9, and 12:10:7, respectively, to prepare hydrogel microspheres. Then, the changes in light transmittance of the emulsion before and after treatment were detected using a UV-Vis spectrophotometer to explore the effects of demulsification and lipid absorption. The dietary fat absorption of the hydrogel microspheres was tested by measuring their ability to absorb fat from emulsified fat.

The specific method was as follows: Soybean oil was used as a fat model, mixed with artificial bile acid and simulated intestinal fluid in a ratio of 1:2:99, and stirred at 2000 rpm for 1 h to prepare emulsified fat. Then, the hydrogel microspheres were placed in the emulsified fat at 37 °C and shaken at 100 rpm for 2 h. The transmittance (T) of the emulsified fat before and after treatment was observed using a UV-Vis spectrophotometer (UV), the fat content in the emulsified fat was observed using a microscope, and the lipid absorption capacity was evaluated by the weight difference of the hydrogel microspheres.

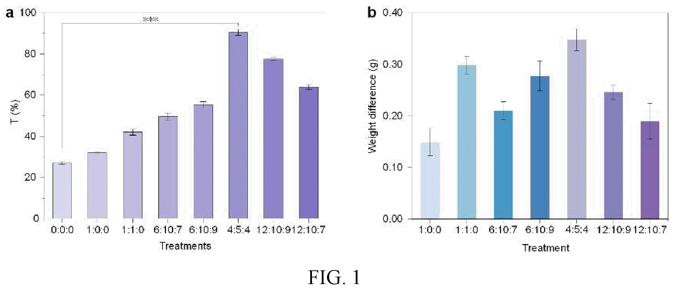

The results are shown in FIG. 1, where (a) is the emulsion light transmittance, and the transmittance from left to right is 27.05±0.73, 32.03±0.59, 42.10±1.43, 49.60±1.66, 55.35±1.42, 89.15±1.20, 77.60±0.71, 63.93±1.26 (unit: %); (b) is the weight difference of the hydrogel microspheres before and after lipid absorption, and the weight difference from left to right is 0.149±0.027, 0.298±0.017, 0.210±0.017, 0.277±0.029, 0.347±0.021, 0.246±0.014, 0.190±0.034 (unit: g).

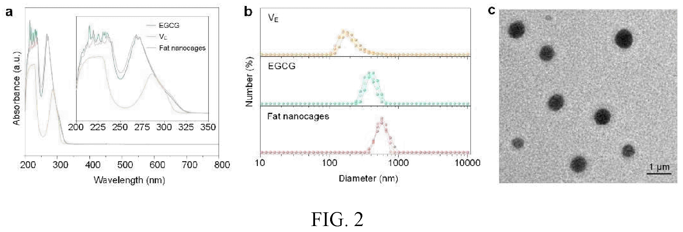

The structure of the hydrogel microspheres prepared with a mass ratio of sodium alginate:EGCG:VE of 4:5:4 was detected. The results are shown in FIG. 2, where (a) is the UV spectrum obtained by UV-Vis spectrophotometer, (b) is the dynamic light scattering (DLS) particle size obtained by a dynamic light scattering instrument, and (c) is a transmission electron microscopy (TEM) image of the hydrophobic cavities. In FIG. 2(b), the EGCG particle size is 379.83±11.77 nm, the VE particle size is 171.23±7.72 nm, and the Fat lock particle size is 574±32.54 nm. As seen from FIG. 2, the hydrogel microspheres prepared by the present disclosure possess hydrophobic cavities.

### Example 2: Preparation and performance testing of hydrogel microspheres

### 2.1 Preparation of hydrogel microspheres

1 g of EGCG and 0.8 g of Vitamin E were dissolved in 3 mL of absolute ethanol solution and shaken at 200 rpm for 30 min to form hydrophobic polyphenol-based micelles. Subsequently, a 2% w/v sodium alginate solution and a 2% w/v calcium sodium edetate solution were mixed by magnetic stirring at a volume ratio of 4:1 to form a uniform mixed solution. Next, the hydrophobic polyphenol-based micelles were added dropwise to the mixed solution to form a uniform pre-gel. Then, the pre-gel was added dropwise into 100 mL of a 2% w/v calcium chloride (CaCl₂) aqueous solution using a constant flow pump and a thin flexible tube (3 cm³), with continuous gentle stirring at 300 rpm. The formed hydrogel microsphere intermediates were solidified in the CaCl₂ aqueous solution for 4 h. Subsequently, the intermediates were immersed in a 0.1 mol/L hydrochloric acid (HCl) solution for 1 h to allow EDTA-Ca to release Ca²⁺ for cross-linking the pre-gel inside the microspheres. Finally, the microspheres were rinsed several times with distilled water to remove residual ions and lyophilized at -80 °C for 48 h to collect the obtained hydrogel microspheres (PFAT).

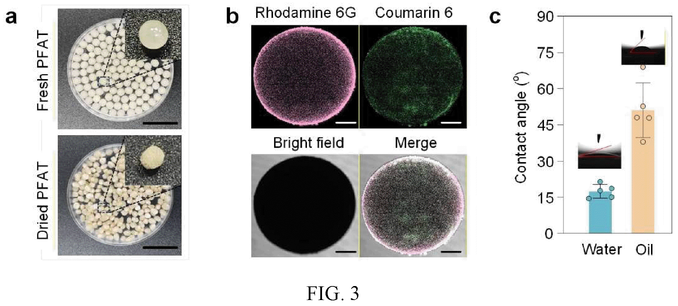

Contact angle detection was performed on the PFAT hydrogel microspheres, and the amphiphilic interface was photographed using a fluorescence microscope. The results are shown in FIG. 3. The results show that the prepared hydrogel microspheres possess amphiphilicity, highlighting the potential for demulsification and lipid absorption. In FIG. 3, (a) shows the macroscopic morphology before lyophilization (Fresh PFAT) and after lyophilization (Dried PFAT); (b) shows the amphiphilic interface of the hydrogel microspheres captured by fluorescence microscopy: pink represents the alginate matrix (hydrophilic), green represents the hydrophobic cavities (hydrophobic), bright-field image, and the merged image showing both red and green; (c) shows the amphiphilicity results of the contact angle test, where the water contact angle is 17.5° and the oil contact angle is 51.1°.

### 2.2 Characterization of hydrogel microspheres

The hydrogel microspheres were analyzed by Fourier-transform infrared spectroscopy (FTIR), organic elemental analysis (EA), and thermogravimetric analysis (TGA). Specifically, the hydrogel microspheres were thoroughly mixed with potassium bromide (KBr) at a ratio of 1:100 w/w and then pressed into transparent pellets. The FTIR spectra of the samples were scanned in the wavelength range of 400-4000 cm⁻¹ at 25 °C. After 5 mg of the hydrogel microspheres were thoroughly ground, the TGA curve was tested in a temperature range of 30-900 °C under a nitrogen environment with a flow rate of 40 mL/min and a heating rate of 10 °C/min. To quantify the content of carbon (C) and hydrogen (H), the samples were completely burned in an oxidation tube at 1150 °C in a pure oxygen environment. To analyze oxygen (O) content, the hydrogel microspheres were pyrolyzed in a mixed gas stream of hydrogen (H₂) and helium (He) at 1150 °C, followed by thermal conductivity detection to determine the concentrations of C, H, and O.

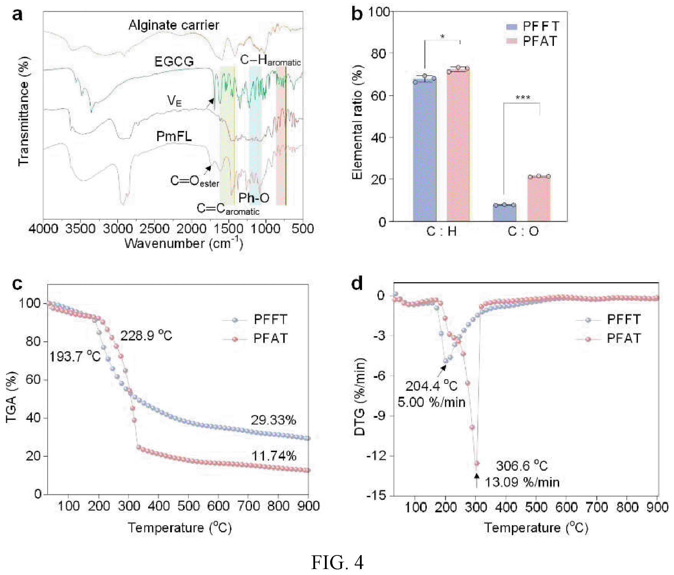

The results show that the polyphenol cavity micelles were successfully filled into the dietary matrix to construct the hydrogel microspheres, as shown in FIG. 4.

In FIG. 4, (a) is the FTIR spectrum of the hydrogel microspheres, used to indicate the composition of the microspheres. Each component has specific peaks, and the final microspheres contain the characteristic peaks of sodium alginate/EGCG/VE, indicating the successful preparation of the hydrogel microspheres; (b) is the elemental analysis diagram, where PFFT represents microspheres prepared using pure sodium alginate in 2.1, and PFAT represents the hydrogel microspheres prepared in 2.1. The change in elemental ratio indicates that the hydrophobic polyphenol-based micelles were successfully filled into the sodium alginate matrix; (c) and (d) are TGA curves. The temperature changes during the decomposition of PFFT (pure sodium alginate microspheres) and PFAT (sodium alginate + EGCG micelle microspheres) indicate the difference in material composition, confirming that the hydrophobic polyphenol-based micelles were successfully incorporated.

### 2.3 Biosafety assessment of hydrogel microspheres

NIH3T3 fibroblast cells were used as the research object to evaluate the biosafety of the hydrogel microspheres through cell viability and cell proliferation. The cytotoxicity of the hydrogel microspheres prepared in 2.1 was evaluated by the Cell Counting Kit-8 (CCK-8) assay. The effect of the hydrogel microspheres on cell proliferation was evaluated using the Annexin V-FITC apoptosis detection kit and confocal laser scanning microscopy (CLSM).

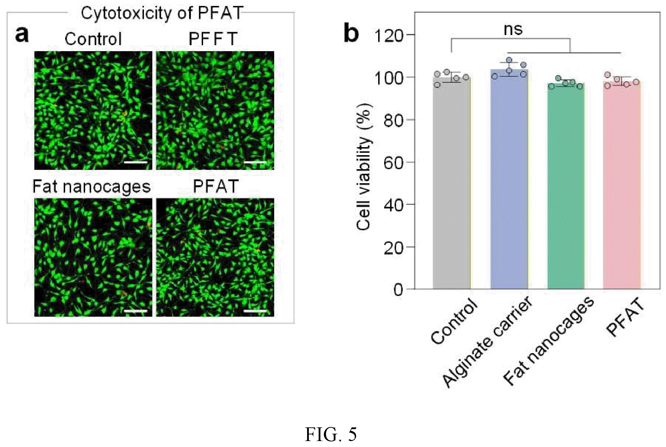

The results are shown in FIG. 5, where (a) is the live/dead fluorescence staining of cells, with green representing live cells and red representing dead cells. PBS was used as the control group, and "Fat locks" represents the polyphenol-based micelles. The results show no visible difference in red/green color compared to the control, qualitatively indicating that the cytotoxicity of the hydrogel is negligible. (b) shows the results of cell viability by CCK-8 assay for quantitative analysis of safety. "Alginate matrix" represents sodium alginate, and the cell viability values from left to right are 100.00±2.26, 103.71±3.17, 97.18±1.60, and 98.18±2.05. The results show no significant difference between the treatment groups and the control group, indicating that the hydrogel microspheres have no significant effect on cell viability.

### 2.4 Gastrointestinal stability testing of hydrogel microspheres

50 mg of the hydrogel microspheres prepared in 2.1 were sequentially immersed in simulated salivary fluid (SSF), simulated gastric fluid (SGF), and simulated intestinal fluid (SIF) for 10 min, 2 h, and 2 h, respectively, with shaking at 200 rpm at 37 °C. After the reaction, the hydrogel microspheres were separated, and 1 mL each of SSF, SGF, and SIF were filtered through a 0.22 µm membrane for analysis.

EGCG and VE in the simulated fluids were quantitatively determined by high-performance liquid chromatography (HPLC). HPLC analysis was performed using a CAPCELL PAK HPLC column (C18, 250 mm × 4.6 mm, 5 µm) equipped with a UV detector. Chromatographic conditions were set at a flow rate of 1 mL/min, an injection volume of 10 µL, and a column temperature of 35 °C. The mobile phases for EGCG and VE were acetonitrile:0.2% acetic acid = 3:7 and 100% methanol, respectively. The detection wavelengths for EGCG and VE were 278 nm and 294 nm, respectively.

Scanning electron microscopy (SEM) was used to evaluate the morphological changes of the hydrogel microspheres before and after the reaction, and the mass loss of the hydrogel microspheres in various fluids was quantitatively evaluated.

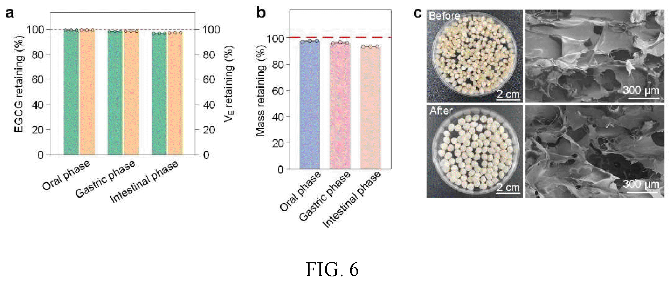

The results are shown in FIG. 6, where (a) shows the EGCG/VE content in the supernatant after immersion in simulated gastrointestinal fluids, indicating the digestion stability of the microspheres in the gastrointestinal tract. The residual amounts of EGCG in SSF/SGF/SIF were 99.32±0.08, 98.46±0.06, and 96.91±0.09, respectively, and the residual amounts of VE in SSF/SGF/SIF were 99.39±0.05, 98.67±0.05, and 97.41±0.05, respectively. (b) shows the weight difference of the microspheres after immersion in simulated gastrointestinal fluids, with residual weights of 97.80±0.47, 96.45±0.54, and 93.57±0.18, respectively. (c) shows the structural difference of the microspheres after immersion. The results indicate that the hydrogel microspheres have good stability in the gastrointestinal tract and maintain structural integrity.

### 2.5 Dietary fat absorption capacity testing of hydrogel microspheres

The dietary fat absorption of the hydrogel microspheres was tested by measuring their ability to absorb fat from emulsified fat. The specific method was: First, various lipids (soybean oil, peanut oil, olive oil, lard, butter, and peanut butter) and free fatty acids (oleic acid, linoleic acid, and DHA) were mixed with artificial bile acid and simulated intestinal fluid in a ratio of 1:2:99, and stirred at 2000 rpm for 1 h to prepare emulsified fat. Then, the hydrogel microspheres were placed in the emulsified fat at 37 °C and shaken at 100 rpm for 2 h. The transmittance (T) of the emulsified fat was observed using a UV-Vis spectrophotometer (UV), the fat content was observed using a microscope, and the lipid absorption capacity was evaluated by the weight difference.

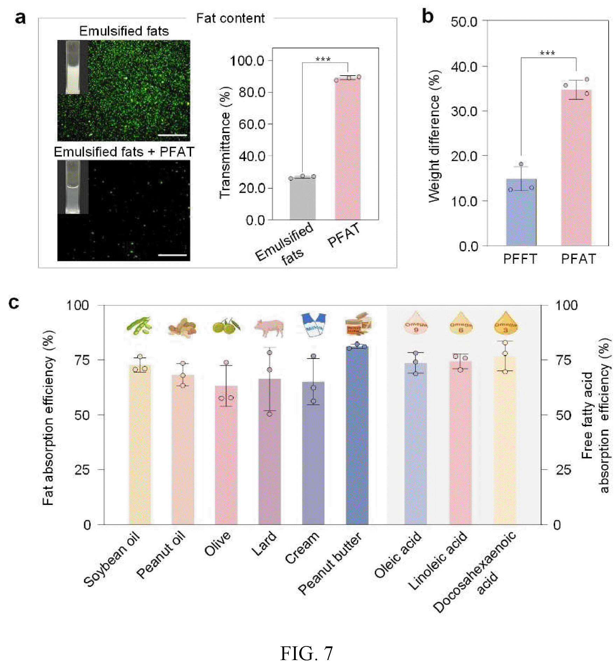

The results are shown in FIG. 7, where (a) shows digital and fluorescence microscopy images of soybean oil, showing the emulsified fat clearly changing from an opaque state to a transparent state, with only sparse coumarin-6-labeled fat droplets visible, indicating effective fat absorption. The weight difference in (b) further shows a reduction in emulsified fat; compared to hydrogel microspheres without polyphenol-based micelles (PFFT), the hydrogel microspheres (PFAT) showed a 19.8% increase in weight gain after fat absorption, demonstrating superior fat absorption capacity. (c) shows that the lipid absorption efficiency of the hydrogel for all dietary lipids was 63.10%-81.13%, with values from left to right in FIG. 7(c) being 72.65±3.31, 68.16±5.05, 63.10±9.27, 66.33±14.36, 65.07±10.49, 81.13±1.01, 73.58±4.70, 74.25±3.36, and 76.74±6.67, proving the exceptional lipid absorption capacity for various dietary fats.

### Example 3: Application of hydrogel microspheres in the preparation of a medicament for preventing intestinal absorption of dietary fats

To study the capacity of hydrogel microspheres to prevent dietary fat absorption in the gastrointestinal tract, 8-week-old male Sprague Dawley (SD) rats of specific pathogen-free (SPF) grade (200±20 g) were used, and soybean oil was labeled with coumarin-6 fluorescence.

Specifically, the rats were divided into three treatment groups: oral administration of 1 mL soybean oil (Fat); oral administration of 1 mL soybean oil and 50 mg Orlistat (Fat+orlistat); and oral administration of 1 mL soybean oil and 100 mg of the hydrogel microspheres prepared in Example 2 (Fat+PFAT). After oral administration, blood samples were taken from the tail vein every 2 h up to 12 h. The collected blood samples were centrifuged at 3000 rpm for 15 min, and then the plasma fat and free fatty acid contents were analyzed using corresponding detection kits. To explore the biodeposition of oil in the rat intestines, the rats were euthanized at two different time points (6 h and 16 h) after oral administration, their gastrointestinal tracts were excised, and fluorescence imaging was performed using IVIS with excitation and emission wavelengths of 465 nm and 520 nm, respectively.

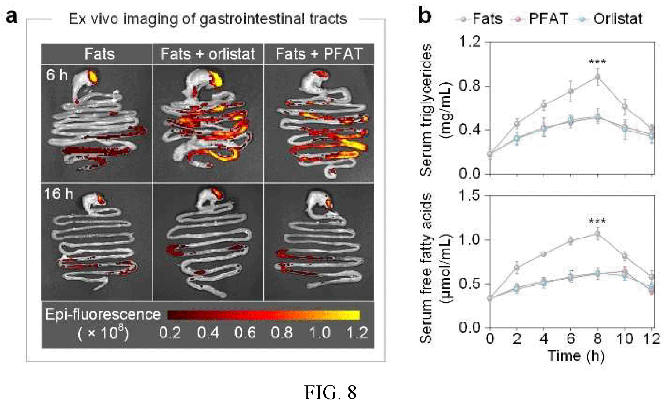

The results are shown in FIG. 8, where (a) is the fluorescence imaging of the gastrointestinal tract and (b) is the content of triglycerides and free fatty acids in the blood. The results show that the hydrogel microspheres effectively prevented the absorption of dietary fats in the gastrointestinal tract.

### Example 4: Application of hydrogel microspheres in the preparation of a medicament for treating obesity

### 4.1 Efficacy testing of hydrogel microspheres in obesity management

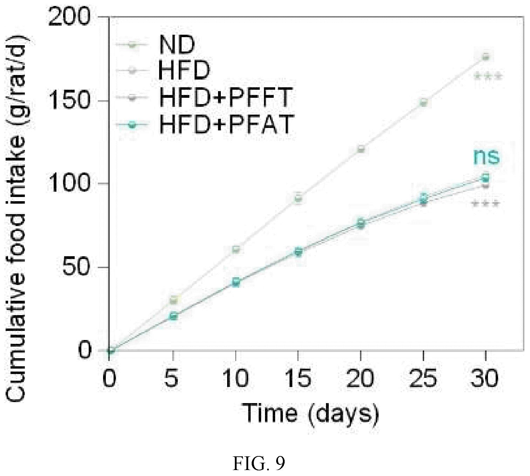

Based on the excellent in vitro absorption capacity, the therapeutic potential of the hydrogel microspheres in obesity treatment was further evaluated using rats fed for 30 d. Male Sprague Dawley (SD) rats were fed a high-fat diet and administered oral hydrogel microspheres for 30 d, after which body weight-related indicators were measured. The rats were randomly divided into four groups: normal diet group (ND), high-fat diet group (HFD), high-fat diet group with hydrogel microspheres prepared in Example 2 (HFD + PFAT), and high-fat diet group with traditional hydrogel (i.e., prepared using only the sodium alginate from Example 2) (HFD + PFFT). During the observation period, the body weight of each rat was recorded every 5 d, and they were fed once daily. The food intake is shown in FIG. 9. After 30 d of feeding, the rats were fasted for 16 h, and then blood was drawn from the abdominal aorta to analyze fat content. Adipose tissues were collected and weighed, and major organs (heart, liver, spleen, lung, kidney, stomach, and intestine) were collected and sectioned for hematoxylin and eosin (H&E) staining; specifically, the liver was weighed and measured. In FIG. 9, the food intake for ND on days 5, 10, 15, 20, 25, and 30 was 30.18±2.43, 60.80±1.99, 91.26±3.58, 120.93±1.77, 149.02±2.23, and 176.18±1.82 g, respectively; for the HFD group, it was 20.68±1.39, 41.06±2.84, 60.00±1.75, 77.21±2.39, 92.24±2.18, and 105.26±1.74 g; for the HFD + PFAT group, it was 20.85±2.21, 41.24±2.17, 59.74±1.00, 76.51±2.00, 90.97±2.65, and 103.70±1.81 g; and for the HFD + PFFT group, it was 20.38±1.98, 40.77±1.99, 58.56±2.42, 74.75±1.81, 88.63±2.30, and 99.18±1.75 g.

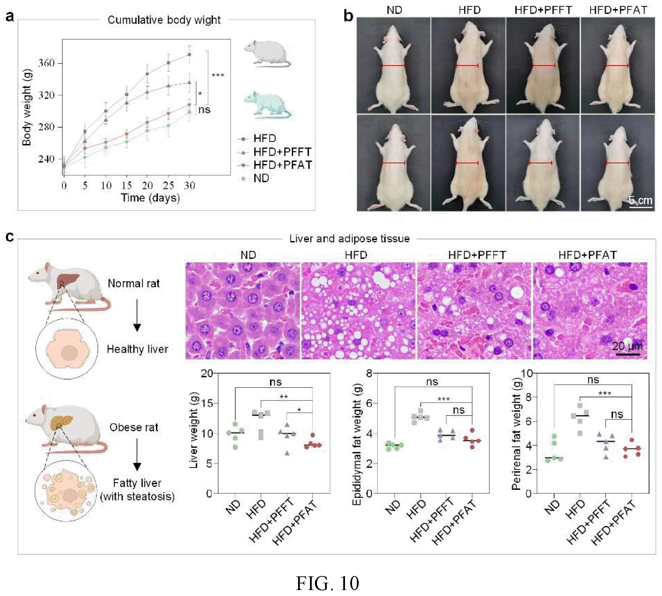

The results are shown in FIG. 10, where (a) is the body weight change of rats in different treatment groups after 30 d. Body weight analysis showed that the weight increase in the HFD + PFFT and HFD + PFAT groups was 90.90±7.74 g (40.78%) and 77.34±7.12 g (33.49%), respectively, which was lower than the 116.42±11.06 g (50.51%) of the HFD group. The weight gain of the ND group was comparable to the HFD + PFAT group at 69.14±10.91 g (30.10%). These results indicate that the administration of hydrogel microspheres reduced weight gain in the HFD group by 17.02%, nearly consistent with the weight change in the ND group. (b) shows the morphology of rats after 30 d; morphologically, the HFD + PFAT rats were very similar to the ND rats and significantly slimmer than the HFD-only rats. (c) shows the weights of the liver and adipose tissues, which are typical obesity indicators. H&E staining sections of liver tissue showed that lipid droplets (white circles) in the HFD + PFAT group were significantly fewer than in the HFD group, indicating reduced liver damage. The data in (c) from left to right show weights for liver, epididymal adipose tissue, and perirenal adipose tissue. Comparative studies found that compared to the HFD group (12.69±1.80 g, 5.08±0.27 g, 6.30±0.85 g), the HFD + PFAT group had lower weights (9.59±1.66 g, 3.59±0.41 g, 3.54±0.56 g), which were similar to the ND group levels (9.83±1.44 g, 3.16±0.17 g, 3.50±0.92 g).

### 4.2 Testing the in vivo excretion capacity of dietary fats by hydrogel microspheres

To address the key challenge of completely inhibiting intestinal absorption and subsequent disposal of fat in obesity treatment, the present disclosure closely monitored and collected rat feces after the 30 d dietary intervention in 4.1. The excretion capacity was tested by observing fecal morphology, lipid content, and water content.

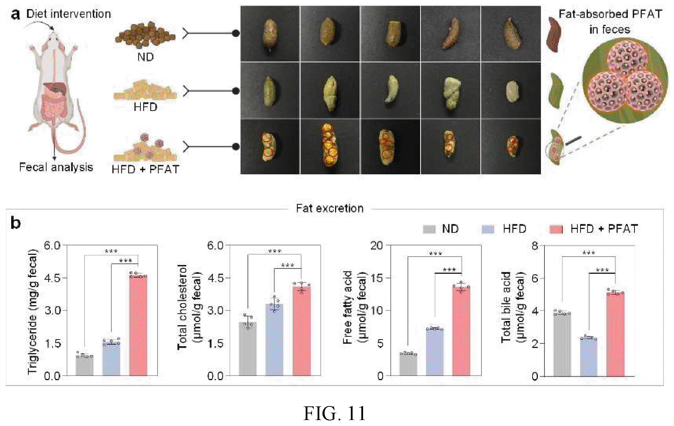

The results are shown in FIG. 11. In FIG. 11a, the feces of the ND group were brown and slightly dry, while the HFD group feces were grass-green and soft. Notably, the feces of the group administered hydrogel microspheres showed distinct yellow particles, indicating that the fat-absorbed hydrogels were excreted. To verify this, fecal lipid levels were analyzed (FIG. 11b). The four charts in (b) show the contents of triglycerides, cholesterol, free fatty acids, and bile acids. Compared with rats consuming only HFD, those in the HFD + PFAT group showed increased fecal levels of these lipids, quantitatively revealing that dietary fats were carried out of the body by the hydrogel microspheres.

### Comparative Example

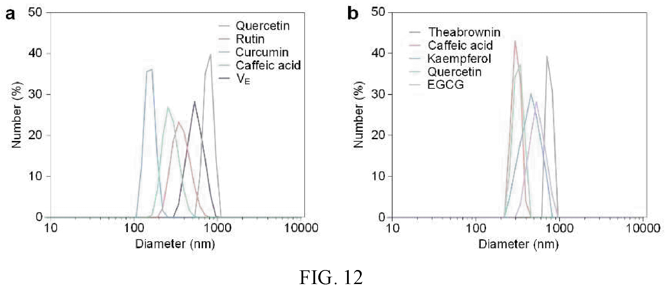

Several different phenols were provided to replace the VE in Example 1 to prepare hydrophobic cavities. The particle sizes of micelles formed by Quercetin, Rutin, Curcumin, Caffeic acid, and VE respectively with EGCG are shown in FIG. 12a, being 825.4±24.3 nm, 342.6±11.7 nm, 164.6±7.5 nm, 255.4±9.7 nm, and 574±32.5 nm, respectively. Comparing the particle sizes in FIG. 12a and FIG. 2 proves that only the reaction of EGCG with VE can produce specific hydrophobic micelles, and the prepared hydrogel microspheres have hydrophobic cavities.

Several different phenols were provided to replace the EGCG in Example 1 to prepare microspheres. Phenols were reacted with VE to obtain micelles. The particle sizes are shown in FIG. 12b: Theabrownin, Caffeic acid, Kaempferol, Quercetin, and EGCG respectively with VE formed micelles with sizes of 712.4±39.3 nm, 295.3±15.6 nm, 458.7±26.5 nm, 336.5±17.4 nm, and 574±32.5 nm.
The above descriptions are only preferred embodiments of the present disclosure and are not intended to limit the present disclosure. For those skilled in the art, various modifications and changes can be made to the present disclosure. Any modification, equivalent replacement, or improvement made within the spirit and principle of the present disclosure shall be included within the scope of protection of the present disclosure.

## Claims

1. A method for preparing hydrogel microspheres, comprising: (a) self-assembling epigallocatechin gallate (EGCG) and Vitamin E in an ethanol solution to form hydrophobic polyphenol-based micelles; (b) uniformly mixing the hydrophobic polyphenol-based micelles with a sodium alginate solution to form a pre-gel; and (c) cross-linking the pre-gel with calcium ions, followed by washing and lyophilization.

2. The method of claim 1, wherein a mass ratio of the epigallocatechin gallate, the Vitamin E, and the sodium alginate is 1 : (0.7-0.9) : (0.6-1.2).

3. The method of claim 1, wherein the sodium alginate solution has a mass concentration of 1.5% to 3%.

4. The method of claim 1, wherein the lyophilization is performed at -80 °C for 36 to 48 hours.

5. The method of claim 1, wherein a concentration of the calcium ions is 1.5% to 2%.

6. A hydrogel microsphere produced by the method according to claim 1.

7. A method for inhibiting intestinal absorption of dietary fats, comprising administering to a subject in need thereof an effective amount of the hydrogel microsphere of claim 6.

8. A method for treating obesity, comprising administering to a subject in need thereof an effective amount of the hydrogel microsphere of claim 6.
